(19) Europäisches Patentamt

**European Patent Office**

**Office européen des brevets**

(11) Veröffentlichungsnummer: **0 254 189 B1**

(12) **EUROPÄISCHE PATENTSCHRIFT**

(45) Veröffentlichungstag der Patentschrift: **04.03.92**

(21) Anmeldenummer: **87110188.7**

(22) Anmeldetag: **15.07.87**

(51) Int. Cl.⁵: **C07C 29/136**, C07C 31/125, C07C 29/17

(54) **Verfahren zur Direkthydrierung von Glyceridölen.**

(30) Priorität: **23.07.86 DE 3624812**
**13.12.86 DE 3642635**

(43) Veröffentlichungstag der Anmeldung:
**27.01.88 Patentblatt 88/04**

(45) Bekanntmachung des Hinweises auf die
Patenterteilung:
**04.03.92 Patentblatt 92/10**

(84) Benannte Vertragsstaaten:
**BE DE FR GB IT NL**

(56) Entgegenhaltungen:
**FR-A- 1 276 722**
**US-A- 2 109 844**
**US-A- 3 180 898**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Carduck, Franz-Josef, Dr.
Landstrasse 18
W-5657 Haan(DE)**
Erfinder: **Falbe, Jürgen, Prof. Dr.
Linnéplatz 14
W-4040 Neuss 1(DE)**
Erfinder: **Fleckenstein, Theo, Dr.
Pfitznerstrasse 9
W-4010 Hilden(DE)**
Erfinder: **Joachim, Pohl, Dr.
Leinenweberweg 23
W-4000 Düsseldorf(DE)**

EP 0 254 189 B1

## Beschreibung

Die Erfindung betrifft ein neues Verfahren zur direkten katalytischen Hydrierung von Glyceridölen unter Verwendung stückiger und/oder gekörnter Kupferchromit enthaltender Katalysatoren.

Im Sinn der vorliegenden Erfindung werden unter nichtentsäuerten Glyceridölen Triglyceridgemische nativer Herkunft verstanden, die neben wenig Mono- und Diglyceriden insbesondere freie Fettsäuren als Verunreinigungen enthalten. Entsäuerte Glyceridöle werden aus den vorstehend beschriebenen Triglyceridgemischen durch Abtrennung der freien Fettsäuren erhalten.

Die direkte katalytische Hydrierung von Glyceridölen ist in der Literatur schon seit langem bekannt. Derartige Prozesse gestatten es, von Fetten und Ölen natürlicher Herkunft auf direktem Weg zu den technisch äußerst interessanten Fettalkoholen zu kommen. Bei der großtechnischen Herstellung von Fettalkoholen wird die Direkthydrierung jedoch bis heute nicht in nennenswertem Umfang angewandt. Man zieht es hier vielmehr vor, die Glyceride der nativen Fette und Öle zunächst mit niederen Monoalkoholen, vorzugsweise mit Methanol, umzuestern, und dann die dabei erhaltenen Fettsäureester niederer Alkohole der katalytischen Hydrierung zu Fettalkoholen zu unterwerfen. Das das auf diese Weise in hohen Ausbeuten und hoher Reinheit gewonnene wertvolle Glycerin gewährleistet eine hohe Wirtschaftlichkeit des zweistufigen Verfahrens.

Aus dem Stand der Technik ist es bekannt, daß bei dem einstufigen Verfahren der Direkthydrierung von Triglyceriden nicht, wie erwartet, Glycerin, sondern u.a. Wasser und Propanol als Nebenprodukte entstehen (W.Normann, Z. angewandte Chemie, 44 (1931), 714-717). Dies wird darauf zurückgeführt, daß das primär gebildete Glycerin bei den vorherrschenden Reaktionsbedingungen unter Wasserabspaltung Folgereaktionen eingeht. Die Tatsache, daß bei der direkten katalytischen Hydrierung von Glyceridölen nach den bekannten Verfahren nicht oder nicht überwiegend hochwertiges Glycerin, sondern weniger wertvolle Nebenprodukte anfallen, hat zur Folge, daß diese Art der Fettalkoholherstellung in der Wirtschaftlichkeit nicht mit dem Zweistufenverfahren konkurrieren kann. Das ist auch der Grund dafür, daß die bekanntgewordenen Verfahren zur Direkthydrierung von Glyceridölen keinen Eingang in die industrielle Praxis gefunden haben.

Verfahren zur Direkthydrierung von Triglyceriden zu höheren aliphatischen Alkoholen wurden auch in der Patentliteratur, beispielsweise in den US-PSen 2 094 127, 2 109 844 und 2 241 417 beschrieben. Die in diesen Druckschriften beanspruchten Verfahren, die bei Wasserstoffdrucken im Bereich von 100 bis 300 bar und Reaktionstemperaturen im Bereich von 200 bis 400°C durchgeführt wurden, ergeben neben den in der Länge der Alkylreste der C-Zahl der Fettsäurereste entsprechenden Fettalkohlen nur geringe Mengen des als Reaktionsprodukt erwünschten Glycerins, dafür größere Mengen an Propan, Propanol oder Propandiolen.

In der DE-OS 16 68 219 wird ein Verfahren zur Hydrierung von Triglyceriden aus Fetten und Ölen beschrieben, in dem mit Eisen, Cobalt, Molybdän, Chrom, Wolfram oder Nickel promotierte Aluminiumoxid- und/oder Siliciumdioxid-Katalysatoren zur Hydrierung bei Temperaturen zwischen 300 und 460°C und Drucken bis 130 bar eingesetzt werden. Auch in dieser Druckschrift wird auf die Gefahr unkontrollierter Folgereaktionen unter Entstehung von Propylenglykol, Propanol oder Propan anstelle des wertvollen Glycerins hingewiesen. Die kontrollierte Reaktionsführung bei extrem hohen Reaktionstemperaturen und -drucken wird angewandt, um vergleichsweise inaktive, dafür aber gegen Katalysatorgift unempfindliche Katalysatoren zur Hydrierung zu verwenden und trotzdem Glycerin als Nebenprodukt erhalten zu können. Für die erwünschten Zwecke wird die Verwendung von Kupferchromit-Katalysatoren ausdrücklich als ungeeignet hingestellt. Ein Nachteil dieses Verfahrens ist darin zu sehen, daß eine selektive Produktsteuerung zu Glycerin als Nebenprodukt nur in einem sehr engen, dafür im Niveau sehr hohen Temperatur- und Druckbereich möglich ist. Bei den genannten Bedingungen wird in der Praxis auch eine Reduktion der erzeugten Fettalkohole zu den entsprechenden Kohlenwasserstoffen beobachtet, die die Wirtschaftlichkeit des Verfahrens aufgrund einer Ausbeuteminderung in Frage stellt. Ein weiterer Nachteil ist darin zu sehen, daß die verwendeten Katalysatoren gegenüber Säurespuren in den aus nativen Quellen zugänglichen Ausgangsstoffen nicht stabil sind und außerdem eine nur ungenügende mechanische Festigkeit aufweisen, so daß nicht nur die Abtrennung des festen Katalysators von den Reaktionsprodukten ausgesprochen schwierig ist, sondern auch durch Abschwemmen von Katalysatormaterial Verluste an aktivem Katalysator in Kauf genommen werden müssen; das kontinuierliche Nachladen aktiven Katalysators stellt ebenfalls die Wirtschaftlichkeit des Verfahrens in Frage.

Es wurde nun überraschend gefunden, daß es mit bestimmten, Kupferchromit als Hauptbestandteil enthaltenden Katalysatoren bei vergleichsweise moderaten Reaktionsbedingungen, d.h. auch bei stark verminderten Drucken, insbesondere jedoch mit hoher Aktivität und Selektivität sowie unter langer Standzeit der Katalysatoren möglich ist, die direkte Hydrierung von Glyceridölen zu Fettalkoholen so zu steuern, daß

EP 0 254 189 B1

als wertvolles Nebenprodukt in hoher Ausbeute 1,2-Propandiol entsteht, das für die Herstellung von Alkyd- oder Polyesterharzen sowie für zahlreiche kosmetische und pharmazeutische Zwecke verwendet werden kann.

Aufgabe der vorliegenden Erfindung war es, ein Verfahren zur direkten katalytischen Hydrierung von Glyceridölen unter Verwendung stückiger und/oder gekörnter, Kupferchromit enthaltender Katalysatoren zur Verfügung zu stellen, mit dem Glyceridöle auch bei relativ, niedrigen Drucken ohne Umweg über eine Spaltung oder Umesterung unmittelbar und in hoher Ausbeute zu Fettalkoholen und 1,2-Propandiol umgesetzt werden können. Der für die Umsetzung verwendete heterogene Übergangsmetall-Katalysator sollte mit hoher Aktivität und Selektivität zu den gewünschten Produkten führen, ohne daß Folgereaktionen in nennenswertem Maß zu einer Verminderung der Produktausbeute beitragen. Neben einer Erhöhung der Katalysatorwirksamkeit im Vergleich zu handelsüblichen Katalysatoren sollte jedoch auch eine Verbesserung der Katalysatorstandzeit erreicht werden.

Zusammen mit einer Einstellung schonender Reaktionsbedingungen sollte damit die Wirtschaftlichkeit des Verfahrens im Vergleich zum Stand der Technik verbessert werden.

Die Erfindung betrifft ein Verfahren zur direkten katalytischen Hydrierung von Glyceridölen bei erhöhten Reaktionstemperaturen und Reaktionsdrucken unter Verwendung stückiger und/oder gekörnter, Kupferchromit als Hauptbestandteil enthaltender Katalysatoren, das dadurch gekennzeichnet ist, daß man entsäuerte oder nichtentsäuerte Glyceridöle zusammen mit Wasserstoff bei Drucken von 20 bis 300 bar und Temperaturen von 160 bis 250 ˚ C bei Molverhältnissen von Wasserstoff : Fettsäureresten im Glyceridöleinsatz von 10 : 1 bis 500 : 1 kontinuierlich über Katalysatoren umsetzt, die 30 bis 40 Gew.-% Kupfer, 23 bis 30 Gew.-% Chrom und 1 bis 7 Gew.-% Barium - Gew.-% bezogen jeweils auf oxidische Katalysatormasse - enthalten und die nach dem Calcinieren der die Katalysatormasse bildenden Komponenten mit 1 bis 10 Gew.-% - bezogen auf oxidischen Katalysator - wenigstens eines Binders und 1 bis 10 Gew.-% - bezogen auf oxidischen Katalysator - Graphit zu stückigen und/oder gekörnten Formlingen, die eine spezifische Oberfläche im Bereich von 20 bis 40 m$^2$/g, ein Porenvolumen von 0,1 bis 1 cm$^3$/g, und bei stückigen Formlingen einen Durchmesser von 1 bis 6 mm und eine Länge von 1 bis 6 mm, bei gekörnten Formlingen eine Korngröße von 0,2 bis 6mm aufweisen, umgewandelt und mit Wasserstoff oder einem Wasserstoff enthaltenden Gasgemisch aktiviert worden sind.

Nach dem erfindungsgemäßen Verfahren katalytisch hydrierbare Glyceridöle können nativen oder synthetischen Ursprungs sein. In der Regel werden jedoch solche Glyceridöle direkt hydriert, die aus Fetten und/oder Ölen aus nativen Quellen stammen. Als Ausgangsstoffe für das erfingungsgemäße Verfahren zur Direkthydrierung kommen also die aus tierischen oder pflanzlichen Quellen entstammenden Fette, Trane oder Öle in Frage, in denen einfach oder mehrfach ungesättigte Fettsäuren mit Glycerin verestert sind, wobei die Fettsäurereste gleiche oder unterschiedliche Sättigungsgrade bzw. Längen ihrer Alkylketten aufweisen können. Mit dem erfindungsgemäßen Verfahren können nicht nur einzelne Triglyceride, sondern auch Triglyceridölmischungen direkthydriert werden.

Die genannten Ausgangsstoffe enthalten - sofern aus natürlichen Quellen stammend - mehr oder weniger große Anteile freier Fettsäuren, die sich auf die Aktivität der Katalysatoren aus dem Stand der Technik immer nachteilig auswirkten. Entsprechend dem erfindungsgemäßen Verfahren ist es möglich, entsäuerte und auch nichtentsäuerte Glyceridöle, d.h. solche Glyceridöle, die auch natürliche Anteile freier Fettsäuren enthalten, direkt zu hydrieren, ohne daß die freien Fettsäuren die Aktivität der in dem erfindungsgemäßen Verfahren verwendeten Katalysatoren in irgendeiner Weise nachteilig beeinflussen. Die Möglichkeit, auch nichtentsäuerte Glyceridöle aus nativen Quellen direkt hydrieren zu können, ist einer der wesentlichen Vorteile des erfindungsgemäßen Verfahrens; die direkte Hydrierung nichtentsäuerter Glyceridöle oder ihrer Mischungen stellt eine bevorzugte Ausführungsform des Verfahrens dar. Die aus dem Stand der Technik üblichen, der eigentlichen Hydrierung vorangehenden Verfahrensschritte der Entsäuerung der Glyceridöle oder ihrer Mischungen kann bei Einsatz des erfindungsgemäßen Verfahrens zur direkten katalytischen Hydrierung nativer Glyceridöle entfallen.

Nach dem erfindungsgemäßen Verfahren wird die direkte katalytische Hydrierung der Glyceridöle in Gegenwart eines Katalysators durchgeführt, der - bezogen auf die oxidische Katalysatormasse - 30 bis 40 Gew.-% Kupfer, 23 bis 30 Gew.-% Chrom und 1 bis 7 Gew.-% Barium enthält. Die genannten Metalle liegen nach der Herstellung der Katalysatormassen, die auf an sich aus dem Stand der Technik bekannte Art und Weise erfolgt, in Form ihrer Oxide vor. Die Oxidbildung erfolgt, wie aus dem Stand der Technik bekannt, im Verlauf des sogenannten "Calcinierens", d.h. einer thermischen Zersetzung von Mineralsalzen der jeweiligen Metalle.

In einer bevorzugten Ausführungsform des erfindungsgemäßen Verfahrens setzt man zur direkten Hydrierung der Triglyceride einen Katalysator ein, der, bezogen auf die oxidische Katalysatormasse, zusätzlich 1 bis 10 Gew.-% SiO$_2$ enthält.

3

EP 0 254 189 B1

In einer bevorzugten Ausführungsform des erfindungsgemäßen Verfahrens hydriert man kontinuierlich Glyceridöle nativer Fette und/oder Öle unter Verwendung eines Katalysators, der vorteilhafterweise 32 bis 38 Gew.-% Kupfer, bezogen auf die oxidische Katalysatormasse, enthält. Ebenso kann es bevorzugt sein, jeweils die Menge an Chrom in dem verwendeten Katalysator auf einen Bereich von 26 bis 29 Gew.-%, jeweils die Menge an Barium auf einen Bereich von 1,5 bis 3 Gew.-% oder jeweils die Menge an Silicium auf einen Bereich von 1,5 bis 3 Gew.-%, jeweils bezogen auf die oxidische Katalysatormasse vor der Aktivierung, einzustellen. In einer besonders bevorzugten Ausführungsform wird zur direkten katalytischen Hydrierung der Glyceridöle nativer Fette oder Öle ein Katalysator verwendet, der 32 bis 38 Gew.-% Kupfer, 26 bis 29 Gew.-% Chrom, 1,5 bis 3 Gew.-% Barium, 1,5 bis 3 Gew.-% Silicium und gegebenenfalls 2,5 Gew.-% Mangan, jeweils bezogen auf die oxidische Katalysatormasse vor der Aktivierung, und gegebenenfalls weitere Übergangsmetalle in Form ihrer Oxide enthält. Mit derartigen Katalysatoren lassen sich erhebliche Aktivitätssteigerungen auch bei relativ niedrigen Drucken erzielen. Aus diesem Grunde ist die Verwendung eines derartigen Katalysators in dem erfindungsgemäßen Verfahren als besonders bevorzugt anzusehen.

In einer weiteren bevorzugten Ausführungsform des Verfahrens kann zur direkten katalytischen Hydrierung der Glyceridöle nativer Fette und/oder Öle ein Katalysator verwendet werden, der zusätzlich zu den oben genannten Mengen an Kupfer, Chrom, Barium und Silicium weitere Übergangsmetalle in Form ihrer Oxide enthält. So kann mit Vorteil ein Katalysator eingesetzt werden, der zusätzlich zu den genannten Metallen je 1 bis 5 Gew.-%, bevorzugterweise 2 bis 3 Gew.-%, Mangan, Zirkon und/oder Cer enthält. Dabei ist es möglich, den erfindungsgemäß verwendeten Katalysatoren eines der genannten Übergangsmetalle in Form seiner Oxide oder auch mehrere der genannten Übergangsmetalle in Form ihrer Oxide in beliebigen Mischungen miteinander zuzusetzen. Die Verwendung derartiger, zusätzlich dotierter Katalysatoren in dem erfindungsgemäßen Verfahren führt insbesondere bei der Hydrierung im Rieselbett zu einer erheblichen Erhöhung der Aktivität und Selektivität der Katalysatoren. Insbesondere kann die Ausbeute an 1.2-Propylenglykol bei der Hydrierung deutlich erhöht werden.

Erfindungsgemäße Katalysatoren enthalten 1 bis 10 Gew.-% an Graphit zur verbesserten Verarbeitbarkeit der Granulat- und/oder Extrudatkörper. Vorzugsweise wird eine Menge von 5 Gew.-% Graphit vor der Granulierung dem calcinierten pulverförmigen Material zugesetzt und mit diesem innig vermischt.

Das erfindungsgemäße Verfahren zur direkten katalytischen Hydrierung von Glyceridölen konnte überraschenderweise auch dadurch verbessert werden, daß durch eine Optimierung nicht nur der chemischen Zusammensetzung des Katalysators, die oben beschrieben wurde, sondern auch des physikalischen Aufbaus der Formkörper des Katalysators eine Erhöhung der Katalysatorwirksamkeit im Vergleich zu handelsüblichen Katalysatoren erreicht wurde. Darunter ist zu verstehen, daß der physikalische Aufbau der Katalysator-Formkörper überraschenderweise ebenfalls erhebliche Auswirkungen auf die Aktivität und Selektivität des Katalysators hat.

Erfindungsgemäß wurde eine Verbesserung des Verfahrens dadurch erreicht, daß der verwendete, die oben genannten Metalle in Form ihrer Oxide und Graphit enthaltende Katalysator unter Einsatz von 1 bis 10 Gew.-% eines oder mehrerer Binder in Granulat- oder Extrudatform gebracht wird. Bevorzugt wird dabei die Verwendung von 1 bis 5 Gew.-% eines oder mehrerer Binder. Als solche kommen für diesen Zweck aus dem Stand der Technik bekannte Verbindungen in Frage, wobei in dem erfindungsgemäß verwendeten Katalysator entweder ein oder auch mehrere Binder verwendet werden können. Besonders hat sich der Einsatz eines oder mehrerer Binder aus Polyvinylacetat, Methylmethacrylat und Kieselsäuresol bewährt. Im Gegensatz zu zahlreichen, aus dem Stand der Technik bekannten, schlecht rieselfähigen Katalysatormaterialien konnte für das erfindungsgemäße Verfahren ein Katalysator in Granular- oder Extrudatform zur Verfügung gestellt werden, dessen aufgelockerte, poröse Struktur zur Erhöhung der Aktivität und Selektivität des Katalysators bei der Direkthydrierung auch bei relativ niedrigen Drucken der Glyceridöle insbesondere im Rieselbett in erheblichem Maße beiträgt. Bevorzugt wird als Binder für die Herstellung der Katalysatorgranulate oder Katalysatorextrudate Polyvinylacetat, wobei zur Katalysatorherstellung beispielsweise 10 Gew.-%ige Polyvinylacetat-Suspensionen verwendet werden, die im Handel erhältlich sind. Die calcinierten pulverförmigen Katalysatormaterialien werden nach guter Durchmischung mit derartigen Polyvinylacetat-Suspensionen in kleinen Mengen versetzt und bis zum Beginn des Aufbaus von Agglomeratkörnern gemischt. Im Anschluß daran wird das Agglomerate enthaltende Pulver beispielsweise in einem Lochwalzengranulator zu kleinen Granulaten verdichtet, wobei dieser Prozeß an sich aus dem Stand der Technik bekannt ist. Auf ebenfalls an sich bekannte Weise werden die Granluate getrocknet, wobei sich Restfeuchten von 10 bis 15 % einstellen lassen. Die aus diesem Vorgang resultierenden Granulate werden gesiebt, wobei für das erfindungsgemäße Verfahren Kornfraktionen bestimmter Korngröße ausgesiebt werden. Bei der Anwendung des erfindungsgemäßen Verfahrens zur katalytischen Direkthydrierung von Glyceridölen in der Rieselfahrweise werden mit Vorteil Katalysator-Kornfraktionen eingesetzt, deren Korn-

4

EP 0 254 189 B1

größe im Bereich von 0,2 bis 6 mm, bevorzugt im Bereich von 0,5 bis 4 mm liegt.

Die Katalysatoren können auch in Tablettenform, beispielsweise der Größe 4 x 4 mm, gepreßt werden. Zur Härtung dieser Tabletten werden diese 6 h bei einer Temperatur von 200°C an der Luft getempert. Die nach der BET-Methode (Z. Anal. Chem. 288 (1968), 187-193) bestimmte spezifische Oberfläche betrug 30 ± 10m$^2$/g.

Die in dem erfindungsgemäßen Verfahren zur Direkthydrierung von Glyceridölen verwendbaren granulierten Katalysatoren weisen eine spezifische Oberfläche im Bereich von 20 bis 40 m$^2$/g auf, wobei der bevorzugte Bereich der spezifischen Oberfläche bei 25 bis 30 m$^2$/g liegt. Die beschriebene Art der Vorgranulierung führt zu einer speziellen, aufgelockerten Porenstruktur, die sich in einer Erhöhung des Porennutzungsgrades auswirkt.

Im Verlaufe der Untersuchungen zu dem erfindungsgemäßen Verfahren zur direkten Hydrierung von Glyceridölen hat es sich gezeigt, daß es besonders bevorzugt ist, die Glyceridöle in Gegenwart eines Katalysators mit Wasserstoff umzusetzen, dessen Granulat- oder Extrudatkörper einen Durchmesser von 1 bis 6 mm, bevorzugt von 2 bis 3 mm, und eine Länge von 1 bis 6 mm, bevorzugt 2 bis 4 mm, aufweisen. Derartige Granulat-bzw. Extrudatkörper (Tabletten) zeigen eine ausgezeichnete Aktivität und Selektivität bei der direkten Umsetzung der Glyceridöle mit Wasserstoff zu langkettigen Fettalkoholen und lassen sich zudem ohne Probleme von den Reaktionsprodukten abtrennen. Außerdem sind die mit diesen Katalysatoren erzielbaren Standzeiten deutlich besser als die Standzeiten der aus dem Stand der Technik bekannten Katalysatoren, die zudem den Nachteil aufwiesen, daß sie zum Teil bei der Reaktion zerfielen und dadurch nur unter großen Problemen von den Reaktionsprodukten abtrennbar waren.

Ein weiterer Faktor, der die Aktivität bzw. Selektivität der erfindungsgemäß eingesetzten Katalysatoren stark beeinflußt, ist das Porenvolumen der Katalysator-Formkörper. Es zeigte sich, daß das Porenvolumen der erfindungsgemäß verwendbaren Katalysatoren in einem Optimalbereich liegen muß, um in dem erfindungsgemäßen Verfahren zur Direkthydrierung von Glyceridölen optimale Ergebnisse zu erbringen. In dem erfindungsgemäßen Verfahrens werden metallhaltige Katalysatoren verwendet, deren Porenvolumen im Bereich von 0,1 bis 1,0 cm$^3$/g liegt. Vorteilhafterweise trägt ein Porenvolumen in diesem Bereich ebenfalls zu einer Erhöhung der Aktivität und Selektivität der Hydrierkatalysatoren bei: Sowohl im Rieselbettreaktor als auch im Sumpfphasenreaktor lassen sich hohe Aktivitäten und Selektivitäten erzielen; gleichzeitig wiesen derartige Katalysatoren in dem erfindungsgemäßen Verfahren eine ausgesprochen hohe Standzeit auf und führten nicht zu Problemen bei der Trennung von Katalysator und Reaktionsprodukten.

Die in dem erfindungsgemäßen Verfahren verwendeten Katalysatoren werden üblicherweise vor ihrem Einsatz in der Direkthydrierung von Glyceridölen mit Wasserstoff oder einem Wasserstoff enthaltenden Gasgemisch aktiviert. Aus ökonomischen Gründen wird mit Vorteil zur Aktivierung der Katalysatormassen ein Gasgemisch verwendet, das überwiegend aus einer Stickstoff-/Wasserstoff-Gasmischung besteht. Vorteilhafterweise kann eine derartige Aktivierung - wie aus dem Stand der Technik bekannt - so durchgeführt werden, daß die Katalysatormassen nach der Herstellung im Stickstoffstrom bei erhöhter Temperatur getrocknet werden und dem trocknenden Gas in steigenden Mengen Wasserstoff zur Aktivierung beigemischt wird. Dabei kann der Wasserstoffanteil in dem aktivierenden Gasgemisch im Bereich von 0,1 bis 10 Vol.-% liegen. Die Aktivierung der Katalysatoren kann dabei sowohl in situ als auch in von dem Reaktionsgefäß getrennten Gefäßen durchgeführt werden.

Die Reaktionstemperaturen der eigentlichen Direkthydrierung nichtentsäuerter Glyceridöle nativer Fette und Öle gemäß der vorliegenden Erfindung liegen im Bereich von 160 bis 250°C, bevorzugt im Bereich von 200 bis 230°C. Bei der Temperaturführung der Reaktion ist allgemein zu beachten, daß die direkte Hydrierung der Glyceridöle zu Fettalkoholen und Propylenglykol eine exotherme chemische Reaktion ist. Diese Erniedrigung der Reaktionstemperatur gegenüber dem Stand der Technik trägt zu einer verbesserten Wirtschaftlichkeit des erfindungsgemäßen Verfahrens bei. Bei der Temperaturführung muß also darauf geachtet werden, daß nach "Anspringen" der Reduktion der Glyceridöle die entstehende Reaktionswärme in üblicher Weise abzuführen ist.

Ein bevorzugter Bereich der Reaktionsdrucke beträgt 20 bis 49, 9 bar, während ein anderer bevorzugter Druckbereich von 50 bis 300 bar reicht.

Ein besonders bevorzugter Bereich der Reaktionsdrucke beträgt 30 bis 49,9 bar. Ein weiterer bevorzugter Bereich der Reaktionsdrucke liegt im Bereich von 200 bis 260 bar. Bei diesen Reaktionsdrucken ist eine hohe Aktivität und Selektivität der Katalysatoren sichergestellt, so daß die Raum-/Zeitausbeute für das erfindungsgemäße Verfahren in einem optimalen Bereich liegt.

Das erfindungsgemäße Verfahren zur Direkthydrierung von Glyceridölen nativer Fette und Öle ist außerdem dadurch gekennzeichnet, daß man ein Molverhältnis von Wasserstoff zu Fettsäurerest im Glyceridölsubstrat von 10 : 1 bis 500 : 1 einstellt. Dies bedeutet, daß die Durchsatzmenge an Wasserstoffgas, gemessen in Mol/Stunde, 10- bis 500mal höher liegt als die Durchsatzmenge an Glyceridöl, gemessen

5

in Mol Fettsäurerest/Stunde. Ein bevorzugter Bereich des Umsatzverhältnisses liegt bei 30 : 1 bis 200 : 1, ebenfalls bezogen auf Fettsäurereste im Glyceridölsubstrat.

Die Vorteile des erfindungsgemäßen Verfahrens sind darin zu sehen, daß durch eine Optimierung sowohl der chemischen Zusammensetzung als auch des physikalischen Aufbaus der verwendeten Katalysatoren auf Kupferchromit-Basis die Katalysatorwirksamkeit im Vergleich zu den Katalysatoren aus dem Stand der Technik deutlich verbessert wurde. Insbesondere konnte durche eine Erhöhung des Porenvolumens und des Porennutzungsgrades der Katalysatorformkörper eine verbesserte Aktivität und Selektivität des Katalysators erreicht werden; die Promotierung des Kupferchromits mit Barium- bzw. Siliciumoxid und gegebenenfalls mit weiteren Übergangsmetalloxiden führte zudem zu einer weiteren erheblichen Aktivitätssteigerung. Dadurch bedingt konnten die Reaktionstemperaturen für die Direkthydrierung von Glyceridölen deutlich gesenkt und dadurch eine Verbesserung der Selektivität des Verfahrens, insbesondere hinsichtlich der Ausbeute an 1.2-Propylenglykol als erwünschtem Nebenprodukt, erreicht werden. Durch die Verwendung eines Festigers konnte der Katalysator in stückige Form gebracht und mit Hilfe dieser Formlinge eine weitere Verbesserung der Gebrauchsfähigkeit des Katalysators erzielt werden: Die durch Granulierung oder Extrudierung erhaltenen Katalysator-Formkörper weisen eine deutlich bessere Bruchhärte auf, zerfallen im Rahmen des erfindungsgemäßen Hydrierverfahrens nicht und bereiten keine Probleme bei der Abtrennung der Reaktionsprodukte von den Katalysatormassen.

Die Erfindung wird durch die nachfolgenden Beispiele näher erläutert.

Beispiel 1
Herstellung eines Katalysators:

Bei Temperaturen von 30 bis 90 °C wurden 84,93 g Ba(NO$_3$)$_2$, 3493 g Cu(NO$_3$)$_2$ . 2 H$_2$O, 294,43 g Mn(NO$_3$)$_2$ . 4 H$_2$O und 62,3 g SiO$_2$ in Forme eines 40 Gew.%igen Kieselsäuresols in 9 l entsalztem Wasser unter starkem Rühren gelöst. In einem zweiten Behälter wurden 1639 g CrO$_3$ in 9 l entsalztem Wasser unter den gleichen Bedingungen gelöst und anschließend 3650 g einer 25 %igen Ammoniaklösung hinzugegeben. Danach wurde die Barium, Mangan und Kupfer enthaltende Lösung bei Temperaturen von 30 bis 90 °C in die vorgelegte Ammoniumchromatlösung gepumpt, wobei aus der Lösung ein Gemenge von Bariumchromat, Manganhydroxid, Siliciumhydroxid und Kupferchromat ausfiel. Die Fällung war beendet bei Absinken des pH-Wertes unter 7.

Das Präzipitat wurde in einer Rahmenfilterpresse abfiltriert und mit entsalztem Wasser nitratfrei gewaschen. Der Filterkuchen wurde über Nacht bei 90 bis 120 °C getrocknet und danach in einer Schneidmühle zu grobem Pulver gemahlen. Das resultierende Chromatpulver wurde im Drehrohrofen bei Temperaturen von 300 bis 500 °C thermisch zum Chromit zersetzt ("calciniert"). Das calcinierte, pulverförmige Material hatte folgende chemische Zusammensetzung:
Cu: 36 ± 0,5 %;
Cr: 29 ± 0,5 %;
Mn: 2,5 ± 0,5 %;
Ba: 1,7 ± 0,5 %; und
Si: 1 ± 0,3 %.

Einem Liter des Pulvers wurden 5 Gew.-% Graphit zugesetzt und im Lödige-Mischer 15 min durchmischt. Anschließend wurden 10 Gew.-% einer 40 gew.-%igen Polyvinylacetatsuspension zugegeben und kurz bis zum Aufbaubeginn von Agglomeraten durchgemischt. Im Anschluß daran wurde das Pulver in einem Lochwalzengranulator zu kleinen Granulaten verdichtet, auf eine Restfeuchte von 10 bis 15 % getrocknet und zu einer 0,6- bis 1,2 mm-Kornfraktion gesiebt.

Das Pulver hatte ausgezeichnete Fließeigenschaften und konnte auf einer Rundläufer-Tablettenmaschine zu Tabletten von 3 bis 6 mm Durchmesser und 2 bis 4 mm Höhe verpreßt werden.

Nach der Härtung (6h, 200 °C, an der Luft) resultierten Tabletten mit einer spezifischen Oberfläche (bestimmt nach BET) von 40 ± 10 m$^2$/g und einem Porenvolumen von 0,4 bis 0,6 cm$^3$/g.

Beispiel 2
Herstellung eines Katalysators:

Bei Temperaturen von 30 bis 90 °C wurden 84,9 g Ba(NO$_3$)$_2$ und 3439 g Cu(NO$_3$)$_2$.3 H$_2$O und 62,3 g SiO$_2$ in Form eines 40 Gew.-%igen Kieselsäuresols in 9 l entsalzten Wassers unter starkem Rühren gelöst. In einem weiteren Reaktionsbehälter wurden 1639 g CrO$_3$ in 9 l entsalzten Wassers unter gleichen Bedingungen gelöst und anschließend 3650 g einer 25%igen Ammoniaklösung zugegeben. Danach wurde die Barium und Kupfer enthaltende Lösung bei 70 °C in die vorgelegte Ammoniumchromatlösung gepumpt, wobei aus der Lösung ein Gemenge von Bariumchromat, Siliciumhydroxid und Kupferchromat ausfiel. Die Fällung war beendet bei Absinken des pH-Wertes unter 7. Das Präzipitat wurde in einer Rahmenfilterpresse abfiltriert und mit entsalztem Wasser nitratfrei gewaschen. Der Filterkuchen wurde über Nacht bei 90 bis 120°C getrocknet und danach in einer Schneidmühle zu grobem Pulver gemahlen. Das resultierende Chromatpulver wurde im Drehrohrofen bei Temperaturen von 300 bis 500°C thermisch zum Chromit zersetzt ("calciniert"). Das calcinierte, pulverförmige Material hatte die folgende Zusammensetzung:

Cu: 38 ± 0,5 %;

Cr: 29 ± 0,5 %;

Si: 1 ± 0,3 % und

Ba: 1,9 ± 0,3 %.

Einem Liter des Pulvers wurden 5 Gew.-% Graphit zugesetzt und im Lödige-Mischer 15 min durchmischt. Anschließend wurden 10 Gew.-% einer 40 gew.-%igen Polyvinylacetatsuspension zugegeben und kurz bis zum Aufbaubeginn von Agglomeraten durchgemischt. Im Anschluß daran wurde das Pulver in einem Lochwalzengranulator zu kleinen Granulaten verdichtet, auf eine Restfeuchte von 10 bis 15 % getrocknet und zu einer 0,6- bis 1,2 mm-Kornfraktion gesiebt.

Das Pulver hatte ausgezeichnete Fließeigenschaften und konnte auf einer Rundläufer-Tablettenmaschine zu Tabletten von 3 bis 6 mm Durchmesser und 2 bis 4 mm Höhe verpreßt werden. Nach der Härtung der Tabletten (6 h, 200°C, an der Luft), lagen die nach BET bestimmten spezifischen Oberflächen bei 30 ± 10 $m^2$/g, das Porenvolumen bei 0,2 bis 0,4 $cm^3$/g und die Tablettenbruchhärte bei 3 bis 7 kp pro Tablette.

Beispiele 3 bis 8
– – – – – – – – –

In einem 1 l Rieselreaktor, der mit Katalysatortabletten (Durchmesser 4 mm; Höhe 4 mm) gemäß Beispiel 1 beschickt war, wurde kontinuierlich mit Aktivkohle und Bleicherde behandeltes Kokosöl - Kennzahlen: Verseifungszahl (VZ) 253; Säurezahl (SZ) 1,3; Jodzahl (JZ) 8,7 - im Gleichstrom mit Wasserstoff umgesetzt. Überschüssiger Wasserstoff wurde nach zweistufiger Abscheidung zusammen mit Frischgas im Kreislauf wieder in die Anlage zurückgeführt.

Die Verfahrensparameter sowie die erzielten Ergebnisse sind der nachfolgenden Tabelle 1 zu entnehmen.

Tabelle 1

| | Beispiele | | | | | |
|---|---|---|---|---|---|---|
| | 3 | 4 | 5 | 6 | 7 | 8 |
| Reaktionsdruck (bar) | 100 | 100 | 50 | 30 | 20 | 20 |
| Reaktionstemperatur (°C) | 200 | 240 | 220 | 220 | 220 | 240 |
| LHSV ($l \times l^{-1} \times h^{-1}$) | 0,5 | 1,0 | 0,5 | 0,5 | 0,5 | 0,5 |
| $H_2$ : Substrat (mol × moläquiv.$^{-1}$) | 200 | 100 | 200 | 200 | 200 | 200 |
| Produkt-Verseifungszahl | 1,0 | 1,0 | 0,6 | 1,5 | 44 | 1,1 |
| Produkt-Zusammensetzung (Gew.-%) | | | | | | |
| Fettalkohole | 85,8 | 85,6 | 85,9 | 85,7 | 71,3 | 85,4 |
| 1,2-Propandiol | 10,7 | 5,5 | 8,5 | 9,3 | 7,4 | 2,0 |
| n-/i-Propanol | 0,48 | 4,8 | 2,34 | 1,64 | 1,42 | 7,79 |
| Kohlenwasserstoffe | 0,03 | 0,3 | 0,17 | 0,1 | 0,1 | 0,5 |

Beispiel 9

In einem 8 l Rieselreaktor mit einem Durchmesser von 4,2 cm der mit Katalysatortabletten (Durchmesser 4 mm; Höhe 4 mm) gemäß Beispiel 2 beschickt war, wurde kontinuierlich mit Aktivkohle und

8

Bleicherde behandeltes Kokosöl - Kennzahlen: Verseifungszahl (VZ) 255; Säurezahl (SZ) 16,5; Hydroxylzahl (OHZ) 18,5 - im Gleichstrom mit Wasserstoff umgesetzt. Überschüssiger Wasserstoff wurde nach zweistufiger Abscheidung zusammen mit Frischgas im Kreislauf wieder in die Anlage zurückgeführt. Der Wasserstoffdruck lag bei 260 bar; die Reaktionstemperatur betrug 230°C.

Die Verfahrensparameter sowie die erzielten Ergebnisse sind der nachfolgenden Tabelle 2 zu entnehmen.

## Tabelle 2

| | |
|---|---|
| Reaktionstemperatur (°C) | 230 |
| Reaktionsdruck (bar) | 260 |
| LHSV ($l \times l^{-1} \times h^{-1}$) | 1 |
| $H_2$ : Öl (mol : moläquiv.) | 100 |
| Produkt-Aussehen | wasserklar |
| Produkt-Kennzahlen: VZ | 1,8 |
| SZ | 0,5 |
| Produkt-Zusammensetzung (Gew.-%) | |
| Fettalkohole | 83,7 |
| 1.2-Propandiol | 9,2 |
| $H_2O$ | 2,8 |
| Methanol/Ethanol | 0,5 |
| n-/i-Propanol | 1,5 |
| Kohlenwasserstoffe | 0,2 |

Beispiele 10 bis 14

Mit dem gemäß Beispiel 1 hergestellten Katalysator wurde kontinuierlich in einem 1-l-Rieselbettreaktor (Beispiele 10 bis 13) bzw. in einem 8-l-Rieselbettreaktor (Beispiel 14) unterschiedlich vorbehandeltes Kokosöl katalytisch hydriert. Die Einzelangaben zur Versuchsdurchführung sowie die Versuchsergebnisse sind der nachfolgenden Tabelle 3 zu entnehmen.

## Tabelle 3

| | Bsp. 10 | Bsp. 11 | Bsp. 12 | Bsp. 13 | Bsp. 14 |
|---|---|---|---|---|---|
| Reaktordurchmesser (cm) | 2,5 | 2,5 | 2,5 | 4 | 4,2 |
| Katalysatormenge (ml) | 250 | 250 | 250 | 500 | 8000 |
| K.-Form (mm) | Tabl., 0,8-1,6 | Gran., 0,8-1,6 | Gran.1,0 | Extr.3x3 | Tabl.4x4 |
| Reaktionsdruck (bar) | 250 | 250 | 250 | 250 | 260 |
| Reaktionstemperatur (°C) | 220 | 225 | 220 | 220 | 200 |
| Substrat | Kokosöl (raffin.) | Kokosöl (1) | Kokosöl (raffin.) | Kokosöl (raffin.) | Kokosöl (raffin.) |
| S.-Kennzahlen VZ | 244 | 255 | 255 | 255 | 255 |
| SZ | 0,6 | 16,5 | 0,6 | 0,5 | 0,5 |
| JZ | 10,25 | | | 9,3 | 9,3 |
| OHZ | 2,1 | 7,0 | 2,1 | 2,1 | 2,1 |
| LHSV ($l \times l^{-1} \times h^{-1}$) | 2,0 | 2,0 | 2,0 | 1,0 | 0,5 |
| $H_2$:Substrat (mol/moläquiv.) | 200:1 | 200:1 | 200:1 | 100:1 | 250:1 |
| Strömungsgeschw. $H_2$ (cm.s$^{-1}$) | 3,7 | 3,7 | 3,7 | 0,73 | 12,2 |
| Produkt-Aussehen | wasserklar | wasserklar | wasserklar | wasserklar | wasserklar |
| P.-Kennzahlen VZ | 0,8 | 1,9 | 0,6 | 2,2 | 0,3 |
| SZ | 1 | 1 | 0,3 | 0,25 | 0,2 |
| JZ | 0,1 | | | | |
| OHZ | 408 | | 406 | | 400 |

Fortsetzung

EP 0 254 189 B1

Fortsetzung Tabelle 3

| Produkt-Zusammensetzung (Gew.-%) | Bsp. 10 | Bsp. 11 | Bsp. 12 | Bsp. 13 | Bsp. 14 |
|---|---|---|---|---|---|
| Fettalkohole | 84,1 | 83,5 | 85,1 | 86,4 | 85,2 |
| 1.2-Propandiol | 11,2 | 9,7 | 11,4 | 9,6 | 11,0 |
| $H_2O$ | 2,1 | 2,7 | 2,7 | 2,2 | 2,7 |
| Methanol/Ethanol | 0,5 | 0,5 | 0,1 | 0,5 | 0,5 |
| n-/i-Propanol | 1 | 1,5 | 0,5 | 1,0 | 0,5 |
| Kohlenwasserstoffe | 0,05 | 0,02 | 0,02 | 0,02 | 0,02 |

Anm. (1): mit Aktivkohle/Bleicherde behandelt

Beispiele 15 und 16
----------

Ein pulverförmiger, calcinierter Kupferoxid-Chromoxid-Katalysator der Zusammensetzung
Cu: 32,8 %
Cr: 27,5 % und

Ba: 6,4 %

wurde, wie in Beispiel 2 beschrieben, granuliert. Die spezifische Oberfläche des Katalysators betrug 37,5 m$^2$/g.

Für die Verwendung in Beispiel 15 wurde das Katalysatorgranulat zu Tabletten mit einem Durchmesser und einer Höhe von je 3,0 mm verpresst. Für die Verwendung in Beispiel 16 wurde eine Fraktion mit Korngrößen im Bereich von 0,8 bis 1,6 mm aus dem Granulat ausgesiebt.

Als zu hydrierendes Substrat diente in beiden Fällen ein mit Aktivkohle/Bleicherde behandeltes Kokosöl. Die kontinuierliche Hydrierung erfolgte jeweils in einem 1-Liter-Rieselbettreaktor mit einem Durchmesser von 2,5 cm, wobei der Wasserstoff im Gleichstrom mit dem Substrat geführt wurde. Die weiteren Prozeßparameter und die Versuchsergebnisse sind der nachstehenden Tabelle 4 zu entnehmen.

Tabelle 4

| | Bsp. 15 | Bsp. 16 |
|---|---|---|
| Katalysatormenge (ml) | 2000 | 250 |
| K.-Form (mm) | Tabl.3,0x3,0 | Gran.0,8-1,6 |
| Reaktionsdruck (bar) | 260 | 250 |
| Reaktionstemperatur (°C) | 220 | 220 |
| Substrat | Kokosöl (1) | Kokosöl (1) |
| S.-Kennzahlen VZ | 255 | 255 |
| SZ | 10 | 10 |
| JZ | – | 5,7 |
| OHZ | 6,3 | 6,3 |
| LHSV ($lxl^{-1}xh^{-1}$) | 0,5 | 1,0 |
| $H_2$: Substrat (mol/moläquiv.) | 400:1 | 400:1 |
| Strömungsgeschw. $H_2$ ($cm.s^{-1}$) | 7,2 | 3,7 |
| Produkt-Aussehen | wasserklar | wasserklar |
| P.-Kennzahlen VZ | 4,5 | 0,9 |
| SZ | 0,3 | 0,3 |
| JZ | 0,4 | 0,4 |
| OHZ | 384 | 384 |
| Produkt-Zusammensetzung (Gew.-%) | | |
| Fettalkohole | 84,0 | 84,4 |
| 1.2-Propandiol | 10,5 | 11,4 |
| $H_2O$ | 2,8 | 2,7 |
| Methanol/Ethanol | 0,5 | 0,5 |
| n-/i-Propanol | 1,0 | 0,5 |
| Kohlenwasserstoffe | 0,2 | 0,1 |

(1) Mit Aktivkohle/Bleicherde behandelt

Vergleichsbeispiele 1 bis 3

Zum Vergleich mit den gemäß Beispiel 2 und 1 hergestellten bzw. in den Beispielen 15 und 16 genannten Katalysatoren wurden herkömmliche, kommerziell erhältliche Katalysatoren im Rieselbettreaktor (1 l) auf ihre Aktivität hin überprüft. Die Prozeßparameter sowie die Ergebnisse sind den nachfolgenden Tabellen 5 und 6 zu entnehmen.

Wie sich bei einem Vergleich der Beispiele 9 bis 16 mit den Vergleichsbeispielen 1 bis 3 zeigt, erreicht in allen Vergleichsbeispielen die Menge an erhaltenem 1.2-Propandiol auch nicht annähernd die theoretisch erreichbare Menge. Vielmehr wird ein deutlich erhöhter Anteil an n- bzw. i-Propanol in der Produktmischung gefunden. Außerdem werden die im Zuge der Hydrierung erhaltenen Fettalkohole in erheblichem Umfang zu Kohlenwasserstoffen reduziert. Die herkömmlichen Verfahren zur direkten Hydrierung sind damit dem erfindungsgemäßen Verfahren unter Verwendung der KupferchromitKatalysatoren deutlich unterlegen.

Tabelle 5

|  | Vgl.-Bsp. 1 | Vgl.-Bsp. 2 | Vgl.-Bsp. 3 |
|---|---|---|---|
| Katalysator | A-Kohle/2,5% Re | CuCr/Ba,Zn | Cu/Zn |
| K.-Menge (ml) | 1000 | 970 | 500 |
| K.-Form (mm) | Strangpresslinge | Tabl., 3,13x3,13 | Tabl., 4x4 |
| Reaktionsdruck (bar) | 250 | 200 | 250 |
| Reaktionstemp. (°C) | 250 | 270 | 220 |
| Substrat | Kokosöl(1) | Kokosöl(roh) | Kokosöl (raffin.) |
| S.-Kennzahlen |  |  |  |
| VZ | 253,7 | 253,7 | 245 |
| SZ | 14,1 | 14,1 | 0,5 |
| OHZ | 18,1 | 18,1 | 3,2 |
| LHSV ($l \times l^{-1} \times h^{-1}$) | 0,2 | 0,45 | 1,0 |
| $H_2$:Substrat (mol/moläquiv.) | 560:1 | 440:1 | 100:1 |
| Strömungsgeschw. $H_2$ (cm.s$^{-1}$) | 4,0 | 10,3 | 0,73 |

Anm.: (1) mit Aktivkohle/Bleicherde behandelt

Tabelle 6

| | Vgl.-Bsp. 1 | Vgl.-Bsp. 2 | Vgl.-Bsp. 3 |
|---|---|---|---|
| Produkt-Aussehen | wasserklar/2. wäßrige Phase | wasserklar/2. wäßrige Phase | katalysator-haltig |
| P.-Kennzahlen VZ | 155 | 2,0 | 49 |
| SZ | 3,9 | 1 | 0,3 |
| OHZ | 120 | | |
| P.-Zusammensetzung (Gew.-%) | | | |
| Fettalkohole | 27,6 | 82,3 | 64,2 |
| 1.2-Propandiol | 0,1 | 0,91 | 5,1 |
| $H_2O$ | 0,46 | 4,0 | 2,0 |
| Methanol/Ethanol | 0,5 | 0,53 | 0,5 |
| n-/i-Propanol | 1,2 | 6,98 | 3,2 |
| Kohlenwasserstoffe | 2,46 | 2,63 | 0,04 |

**Patentansprüche**

1. Verfahren zur direkten katalytischen Hydrierung von Glyceridölen bei erhöhten Reaktionstemperaturen und Reaktionsdrucken unter Verwendung stückiger und/oder gekörnter, Kupferchromit als Hauptbestandteil enthaltender Katalysatoren, dadurch gekennzeichnet, daß man entsäuerte oder nichtentsäuerte Glyceridöle zusammen mit Wasserstoff bei Drucken von 20 bis 300 bar und Temperaturen von 160 bis 250° C bei Molverhältnissen von Wasserstoff : Fettsäureresten im Glyceridöleinsatz von 10 : 1 bis 500 : 1 kontinuierlich über Katalysatoren umsetzt, die 30 bis 40 Gew.-% Kupfer, 23 bis 30 Gew.-% Chrom, 1 bis 7 Gew.-% Barium - Gew.-% bezogen jeweils auf oxidische Katalysatormasse - enthalten und die nach dem Calcinieren der die Katalysatormasse bildenden Komponenten mit 1 bis 10 Gew.-% - bezogen auf oxidischen Katalysator - wenigstens eines Binders und 1 bis 10 Gew.-% - bezogen auf

15

oxidische Katalysator - Graphit zu stückigen und/oder gekörnten Formlingen, die eine spezifische Oberfläche im Bereich von 20 bis 40 m²/g, ein Porenvolumen von 0,1 bis 1 cm³/g, und bei stückigen Formlingen einen Durchmesser von 1 bis 6 mm und eine Länge von 1 bis 6 mm, bei gekörnten Formlingen eine Korngröße von 0,2 bis 6 mm aufweisen, umgewandelt und mit Wasserstoff oder einem Wasserstoff enthaltenden Gasgemisch aktiviert worden sind.

2. Verfahren nach Anspruch 1, dadurch gekennzeichnet, daß man bei Drucken von 20 bis 49,9 bar hydriert.

3. Verfahren nach Anspruch 1, dadurch gekennzeichnet, daß man bei Drucken von 50 bis 300 bar hydriert.

4. Verfahren nach Ansprüchen 1 bis 3, dadurch gekennzeichnet, daß der Katalysator, bezogen auf die oxidische Katalysatormasse, zusätzlich 1 bis 10 Gew.-% Silicium enthält.

5. Verfahren nach Ansprüchen 1 bis 4, dadurch gekennzeichnet, daß man kontinuierlich nichtentsäuerte Glyceridöle nativer Fette oder Öle katalytisch mit Wasserstoff umsetzt.

6. Verfahren nach Anspruch 5, dadurch gekennzeichnet, daß der Katalysator 32 bis 38 Gew.-%, bezogen auf die oxidische Katalysatormasse, Kupfer enthält.

7. Verfahren nach Anspruch 5, dadurch gekennzeichnet, daß der Katalysator 26 bis 29 Gew.-%, bezogen auf die oxidische Katalysatormasse, Chrom enthält.

8. Verfahren nach Anspruch 5, dadurch gekennzeichnet, daß der Katalysator 1,5 bis 3 Gew.-%, bezogen auf die oxidische Katalysatormasse, Barium enthält.

9. Verfahren nach Anspruch 5, dadurch gekennzeichnet, daß der Katalysator 1,5 bis 3 Gew.-%, bezogen auf die oxidische Katalysatormasse, Silicium enthält.

10. Verfahren nach Anspruch 5, dadurch gekennzeichnet, daß der Katalysator 32 bis 38 Gew.-%, jeweils bezogen auf die oxidische Katalysatormasse, Kupfer, 26 bis 29 Gew.-% Chrom, 1,5 bis 3 Gew.-% Barium, 1,5 bis 3 Gew.-% Silicium und gegebenenfalls 2,5 Gew.-% Mangan in Form ihrer Oxide enthält.

11. Verfahren nach Anspruch 10, dadurch gekennzeichnet, daß der Katalysator zusätzlich je 1 bis 5, bevorzugt 2 bis 3 Gew.-%, bezogen auf die oxidische Katalysatormasse, Mangan, Zirkon und/oder Cer in Form ihrer Oxide enthält.

12. Verfahren nach Ansprüchen 5 bis 11, dadurch gekennzeichnet, daß der Katalysator unter Einsatz von 1 bis 5 Gew.-%, bezogen auf die oxidische Katalysatormasse, eines oder mehrerer Binder aus Polyvinylacetat, Methylmethacrylat und Kieselsäuresol in Granulat- oder Extrudatform gebracht worden ist.

13. Verfahren nach Ansprüchen 5 bis 12, dadurch gekennzeichnet, daß der Katalysator unter Einsatz von 1 bis 5 Gew.-% an Graphit, bezogen auf die oxidische Katalysatormasse, in Granulatform oder Extrudatform gebracht worden ist.

14. Verfahren nach Ansprüchen 5 bis 13, dadurch gekennzeichnet, daß der Katalysator eine Korngröße im Bereich von 0,5 bis 4 mm aufweist.

15. Verfahren nach Ansprüchen 5 bis 14, dadurch gekennzeichnet, daß die Granulat- oder Extrudatkörper des Katalysators einen Durchmesser von 2 bis 3 mm und eine Länge von 2 bis 4 mm aufweisen.

16. Verfahren nach Ansprüchen 5 bis 15, dadurch gekennzeichnet, daß der Katalysator eine spezifische Oberfläche im Bereich von 25 bis 30 m²/g aufweist.

17. Verfahren nach Ansprüchen 5 bis 16, dadurch gekennzeichnet, daß der Katalysator mit einem 0,1 bis 10 Vol.-% Wasserstoff enthaltenden $N_2$-/$H_2$-Gasgemisch aktiviert worden ist.

**18.** Verfahren nach Ansprüchen 5 bis 17, dadurch gekennzeichnet, daß man bei Reaktionsdrucken von 30 bis 49,9 bar mit Wasserstoff umsetzt.

**19.** Verfahren nach Ansprüchen 5 bis 17, dadurch gekennzeichnet, daß man bei Reaktionsdrucken von 200 bis 260 bar mit Wasserstoff umsetzt.

**20.** Verfahren nach Ansprüchen 5 bis 19, dadurch gekennzeichnet, daß man bei Reaktionstemperaturen von 200 bis 230 °C mit Wasserstoff umsetzt.

**21.** Verfahren nach Ansprüchen 5 bis 20, dadurch gekennzeichnet, daß man ein Verhältnis $H_2$ : Fettsäurerest im Glyceridöl-Substrat von 30 : 1 bis 200 : 1 , bezogen auf einen Fettsäurerest des Glyceridöl-Substrats, einstellt.

## Claims

**1.** A process for the direct catalytic hydrogenation of glyceride oils at high reaction temperatures and under high reaction pressures using particulate and/or granulated catalysts containing copper chromite as principal constituent, characterized in that deacidified or non-deacidified glyceride oils are continuously reacted with hydrogen under pressures of from 20 to 300 bar and at temperatures of from 160 to 250 °C with molar ratios of hydrogen to fatty acid residues in the glyceride oil substrate of from 10:1 to 500:1, the reaction being carried out over catalysts which contain from 30 to 40% by weight copper, from 23 to 30% by weight chromium and from 1 to 7% by weight barium (% by weight, based in each case on oxidic catalyst mass) and which, after calcination of the components forming the catalyst mass, have been converted into shaped particulate and/or coarse-particle elements having a specific surface of 20 to 40 $m^2$/g, a pore volume of 0.1 to 1 $cm^3$/g and - in the case of particulate elements - a diameter of 1 to 6 mm and a length of 1 to 6 mm, in the case of granulated elements a grain size of 0.2 to 6 mm with from 1 to 10% by weight, based on oxidic catalyst, of at least one binder and 1 to 10% by weight, based on oxidic catalyst, of graphite and activated with hydrogen or a hydrogen-containing gas mixture.

**2.** A process as claimed in claim 1, characterized in that the hydrogenation is carried out under pressures of from 20 to 49.9 bar.

**3.** A process as claimed in claim 1, characterized in that the hydrogenation is carried out under pressures of from 50 to 300 bar.

**4.** A process as claimed in claims 1 to 3, characterized in that the catalyst additionally contains from 1 to 10% by weight silicon, based on the oxidic catalyst mass.

**5.** A process as claimed in claims 1 to 4, characterized in that non-deacidified glyceride oils of native fats or oils are continuously reacted catalytically with hydrogen.

**6.** A process as claimed in claim 5, characterized in that the catalyst contains from 32 to 38% by weight copper, based on the oxidic catalyst mass.

**7.** A process as claimed in claim 5, characterized in that the catalyst contains from 26 to 29% by weight chromium, based on the oxidic catalyst mass.

**8.** A process as claimed in claim 5, characterized in that the catalyst contains from 1.5 to 3% by weight barium, based on the oxidic catalyst mass.

**9.** A process as claimed in claim 5, characterized in that the catalyst contains from 1.5 to 3% by weight silicon, based on the oxidic catalyst mass.

**10.** A process as claimed in claim 5, characterized in that the catalyst contains (based in each case on the oxidic catalyst mass) from 32 to 38% by weight copper, from 26 to 29% by weight chromium, from 1.5 to 3% by weight barium, from 1.5 to 3% by weight silicon and, optionally, 2.5% by weight manganese in the form of their oxides.

17

**11.** A process as claimed in claim 10, characterized in that the catalyst contains 1 to 1.5% by weight and preferably from 2 to 3% by weight, based on the oxidic catalyst mass, of manganese, zirconium and/or cerium in the form of their oxides.

**12.** A process as claimed in claims 5 to 11, characterized in that the catalyst has been brought into granule or extrudate form using from 1 to 5% by weight, based on the oxidic catalyst mass, of one or more binders from the group comprising polyvinyl acetate, methyl methacrylate and silica sol.

**13.** A process as claimed in claims 5 to 12, characterized in that the catalyst has been brought into granule or extrudate from using from 1 to 5% by weight of graphite based on the oxidic catalyst mass.

**14.** A process as claimed in claims 5 to 13, characterized in that the catalyst has a grain size of from 0.5 to 4 mm.

**15.** A process as claimed in claims 5 to 14, characterized in that the granules or extrudates of the catalyst have a diameter of from 2 to 3 mm and a length of from 2 to 4 mm.

**16.** A process as claimed in claims 5 to 15, characterized in that the catalyst has a specific surface of from 25 to 30 $m^2/g$.

**17.** A process as claimed in claims 5 to 16, characterized in that the catalyst has been activated with an $N_2/H_2$ gas mixture containing from 0.1 to 10% by volume hydrogen.

**18.** A process as claimed in claims 5 to 17, characterized in that the reaction with hydrogen is carried out under reaction pressures of from 30 to 49.9 bar.

**19.** A process as claimed in claims 5 to 17, characterized in that the reaction with hydrogen is carried out under reaction pressures of from 200 to 260 bar.

**20.** A process as claimed in claims 5 to 19, characterized in that the reaction with hydrogen is carried out at reaction temperatures of from 200 to 230°C.

**21.** A process as claimed in claims 5 to 20, characterized in that a ratio of $H_2$ to fatty acid residue in the glyceride oil substrate of from 30:1 to 200:1, based on one fatty acid residue of the glyceride oil substrate, is established.

**Revendications**

**1.** Procédé d'hydrogénation catalytique directe de glycérides à température de réaction et à pression de réaction élevées, par utilisation de catalyseurs contenant de la chromite de Cuivre en morceaux ou en grains comme constituant principal, procédé caractérisé en ce que l'on met en réaction de glycérides désacidifiées ou non désacidifiées ensemble avec l'hydrogène à des pressions allant de 20 à 300 bars et à des températures de 160 à 250°C pour des rapports molaires hydrogène/radicaux d'acides gras dans l'utilisation d'essai de glycéride allant de 10 : 1 à 500 : 1, en continu sur des catalyseurs qui renferment 30 à 40 % en poids de Cuivre, 23 à 30 % en poids de Chrome, 1à 7 % en poids de Baryum, (les % en poids rapportés respectivement à la masse de catalyseur oxydique) et qui sont convertis après calcination des composants formant la masse de catalyseur avec 1 à 10 % en poids, rapporté au catalyseur oxydique, d'au moins un agent liant et 1 à 10 % en poids, rapporté au catalyseur oxydique, de graphite en des agglomérés en morceaux et/ou en grains, qui possèdent une surface spécifique dans la zone de 20 à 40 $m^2/g$, un volume de pores de 0,1 à 1 $cm^3/g$ et pour les agglomérés en morceaux un diamètre de 1 à 6 mm et une longueur de 1 à 6 mm, pour les agglomérés en grains une taille de grains de 0,2 à 6 mm et qui ont été activés avec de l'hydrogène ou avec un mélange gazeux contenant de l'hydrogène.

**2.** Procédé selon la revendication 1, caractérisé en ce que l'on réalise l'hydrogénation à des pressions allant de 20 à 49,9 bars.

**3.** Procédé selon la revendication 1, caractérisé en ce que l'on réalise l'hydrogénation à des pressions

EP 0 254 189 B1

allant de 50 à 300 bars.

4. Procédé selon les revendications 1 à 3, caractérisé en ce que le catalyseur renferme en supplément de 1 à 10 % en poids de silicium rapporté à la masse de catalyseur oxydique.

5. Procédé selon les revendications 1 à 4, caractérisé en ce que l'on fait réagir catalytiquement d'une manière continue des glycérides non désacidifiées de graisses ou huiles natives, avec de l'hydrogène.

6. Procédé selon la revendication 5, caractérisé en ce que le catalyseur renferme de 32 à 38 % en poids de Cuivre, rapporté à la masse de catalyseur oxydique.

7. Procédé selon la revendication 5, caractérisé en ce que le catalyseur renferme de 26 à 29 % en poids de chrome, rapporté à la masse de catalyseur oxydique.

8. Procédé selon la revendication 5, caractérisé en ce que le catalyseur renferme de 1,5 à 3 % en poids de Baryum, rapporté à la masse de catalyseur oxydique.

9. Procédé selon la revendication 5, caractérisé en ce que le catalyseur renferme de 1,5 à 3 % en poids de silicium, rapporté à la masse de catalyseur oxydique.

10. Procédé selon la revendication 5, caractérisé en ce que le catalyseur renferme 32 à 38 % en poids de Cuivre - respectivement rapporté à) la masse de catalyseur oxydique -, 26 à 29 % en poids de chrome, 1,5 à 3 % en poids de Baryum, 1,5 à 3 % en poids de silicium et éventuellement 2,5 % en poids de Manganèse sous forme de leurs oxydes.

11. Procédé selon la revendication 10, caractérisé en ce que le catalyseur renferme un supplément respectivement de 1 à 5 - de préférence de 2 à 3 % en poids - rapporté à la masse de catalyseur oxydique, de Manganèse, de Zirconium, et/ou de Cérium sous forme de leurs oxydes.

12. Procédé selon les revendications 5 à 11, caractérisé en ce que le catalyseur est amené sous forme de granulé ou d'extrudat par mise en jeu de 1 à 5 % en poids, rapporté à la masse de catalyseur oxydique, d'un ou plusieurs agents liants en acétate de polyvinyle, méthacrylate de méthyle, et sol d'acide silicique.

13. Procédé selon les revendications 5 à 12, caractérisé en ce que le catalyseur est amené sous forme de granulé ou sous forme d'extrudat par mise en jeu de 1 à 5 % en poids de graphite, rapporté à la masse de catalyseur oxydique.

14. Procédé selon les revendications 5 à 13, caractérisé en ce que le catalyseur possède une taille de grains dans la zone de 0,5 à 4 mm.

15. Procédé selon les revendications 5 à 14, caractérisé en ce que les solides granulés ou extrudés de catalyseur possèdent un diamètre de 2 à 3 mm et une longueur de 2 à 4 mm.

16. Procédé selon les revendications 5 à 15, caractérisé en ce que le catalyseur possède une surface spécifique dans la zone allant de 25 à 30 $m^2$/g.

17. Procédé selon les revendications 5 à 16, caractérisé en ce que le catalyseur a été activé par un mélange de gaz $N_2/H_2$ contenant de 0,1 à 10 % en volume d'hydrogène.

18. Procédé selon les revendications 5 à 17, caractérisé en ce que l'on fait réagir avec l'hydrogène à des pressions de réaction allant de 30 à 49,9 bars.

19. Procédé selon les revendications 5 à 17, caractérisé en ce que l'on fait réagir avec l'hydrogène à des pressions de réaction allant de 200 à 260 bars.

20. Procédé selon les revendications 5 à 19, caractérisé en ce que l'on fait réagir avec l'hydrogène à des températures de réaction allant de 200 à 230° C.

19

21. Procédé selon les revendications 5 à 20, caractérisé en ce que l'on ajuste un rapport $H_2$ : reste d'acide gras dans le substrat de glycéride allant de 30 : 1 à 200 : 1, rapporté à un reste d'acide gras du substrat de glycéride.